# NEUE EUROPÄISCHE PATENTSCHRIFT

(11) **EP 3 089 480 B2**
(45) Veröffentlichungstag und Bekanntmachung des Hinweises auf die Entscheidung über den Einspruch: **17.06.2020**
(45) Hinweis auf die Patenterteilung: 26.07.2017
(21) Anmeldenummer: 16164091.7
(22) Anmeldetag: 06.04.2016
(51) Int. Cl.: H04R 25/00

(54) **VERFAHREN ZUM HERSTELLEN EINER OTOPLASTIK UND OTOPLASTIK**
METHOD FOR PRODUCING AN OTOPLASTIC AND OTOPLASTIC
PROCÉDÉ DE FABRICATION D'UNE PROTHÈSE AUDITIVE ET PROTHÈSE AUDITIVE

(30) Priorität: 30.04.2015 AT 503532015
(43) Veröffentlichungstag der Anmeldung: 02.11.2016
(73) Patentinhaber: Audio lab Austria GmbH, 8421 Wolfsberg im Schwarzautal (AT)
(72) Erfinder: Bauer, Markus, 8350 Fehring (AT)
(74) Vertreter: Wirnsberger, Gernot

(56) Entgegenhaltungen:
- EP-A1- 1 246 505
- EP-A1- 1 629 805
- EP-A1- 1 795 160
- EP-A1- 2 615 854
- EP-A2- 0 263 667
- EP-A2- 0 478 892
- EP-A2- 0 590 698
- EP-B1- 0 660 642
- WO-A1-02/03757
- WO-A1-02/071794
- WO-A1-2005/041831
- US-A- 6 144 750
- US-A1- 2011 271 965
- US-A1- 2013 251 161
- US-B2- 6 938 622
- Hörnig, M. et al: "Faceplate cover for ITE Hearing Aid by Thermoforming Technique", Journal Technique, vol. 8, 11 April 2012 (2012-04-11), page 65,

## Beschreibung

Die Erfindung betrifft ein Verfahren zum Herstellen einer Otoplastik, wobei ein digitales dreidimensionales Modell einer Ohrmuschel erstellt wird, gemäß welchem die Otoplastik mit einem 3-D-Drucker gedruckt wird.

Darüber hinaus betrifft die Erfindung eine Otoplastik zum teilweisen Einführen in einen Gehörgang und/oder Tragen in einer Ohrmuschel, wobei die Otoplastik mit einem 3-D-Drucker gedruckt ist.

Weiter betrifft die Erfindung eine Verwendung einer solchen Otoplastik.

Eine Otoplastik ist ein Formpassstück, welches zumindest teilweise in einen Gehörgang eines Menschen einführbar bzw. in einer Ohrmuschel tragbar ist. Bevorzugt wird eine Otoplastik individuell für einen Träger hergestellt, da sich eine Form der Ohren sowie des Gehörganges von Mensch zu Mensch unterscheidet. Dazu wird in einem ersten Schritt der Gehörgang und die Ohrmuschel bzw. der für die Otoplastik benötigte Bereich des Ohres mit einer Abformmasse aus beispielsweise Silicon abgeformt. Nach einer Aushärtung dieses Materials ist ein Positivabdruck des Ohres hergestellt, welcher gescannt und nach welchem ein digitales Modell desselben erstellt wird.

Das digitale Modell wird an einem Computer weiterbearbeitet, wonach die Otoplastik dem Modell entsprechend produziert wird. Dies kann beispielsweise durch ein Übereinanderlegen von einer Vielzahl hauchdünner Kunststoffschichten erfolgen, welche anschließend unter UV-Licht ausgehärtet werden. Als Alternative dazu kann die Otoplastik mittels eines 3-D-Druckers als Ganzes gedruckt werden. Um eine solche Otoplastik in eine endgültige, tragbare Form zu bringen, wird diese zum Abschluss noch von Hand bearbeitet.

Eine Otoplastik wird in unterschiedlichen Anwendungsgebieten eingesetzt, beispielsweise als Gehörschutz, Hörhilfe oder Miniaturkopfhörer. Eine hierzu verwendete Otoplastik ist meistens unifarben, bevorzugt in dunklen oder hautähnlichen Farbtönen ausgebildet. Es ist auch bekannt, eine Otoplastik in unterschiedlichen Farben herzustellen bzw. diese mit beispielsweise Strasssteinen zu verzieren. Dies betrifft jedoch hauptsächlich eine reine Schmuckotoplastik, welche keinen weiteren Zweck erfüllt. Ein einer solchen Schmuckotoplastik entsprechendes Design bzw. Erscheinungsbild ist jedoch nicht ohne Weiteres auf beispielsweise einen Gehörschutz übertragbar. Ein Gehörschutz umfasst technische Mittel wie einen Schallfilter, welche nicht verziert bzw. abgedeckt werden können, ohne deren Zweck zumindest einzuschränken. Zudem ist eine Herstellung einer Otoplastik mit solchen Verzierungen kostenintensiv, insbesondere wenn diese mit einem individuellen Design hergestellt werden soll. Darüber hinaus ist zumindest eine filigran gestaltete Schmuckotoplastik weder besonders langlebig noch angenehm zu tragen. Das Dokument US 2013/251161 zeigt eine Otoplastik, wobei eine Abdeckung aus empfindlichen Materialien hergestellt werden kann und eine Gravierung aufweisen kann. Hier setzt die Erfindung an. Aufgabe der Erfindung ist es, ein Verfahren der eingangs genannten Art anzugeben, mit welchem eine vielfältig einsetzbare Otoplastik in einer einfachen Weise optisch ansprechend hergestellt werden kann.

Ein weiteres Ziel ist es, eine Otoplastik der eingangs genannten Art anzugeben, welche vielfältig einsetzbar und optisch ansprechend ist.

Weiter ist es Ziel, eine Verwendung einer solchen Otoplastik anzugeben.

Die Aufgabe wird erfindungsgemäß durch ein Verfahren gemäß Ansrpuch 1 gelöst. Ein mit der Erfindung erzielter Vorteil ist insbesondere darin zu sehen, dass durch diese zwei relativ einfachen Schritte ein sichtbarer Teil der Otoplastik in einer einfachen Weise optisch ansprechend gestaltet wird. Die Abdeckung kann dabei aus einem beliebigen Material hergestellt werden und deckt die sichtbare Fläche ab. Geeignet ist beispielsweise Holz, Papier, PVC-Folien, Metall und/oder Stoff. Günstig ist es in jedem Fall, wenn die Abdeckung eine geringe Dicke aufweist, insbesondere weniger als 0,5 mm, bevorzugt weniger als 0,3 mm, besonders bevorzugt 0,2 mm oder weniger. Die Abdeckung wird dann auf den äußeren bzw. sichtbaren Bereich bzw. die äußere Fläche der Otoplastik geklebt, welche die Otoplastik nach außen hin abschließt. Die äußere Fläche ist bevorzugt eben bzw. plan und frei von Unebenheiten und/oder Rillen ausgebildet. Um die Abdeckung gegen Abnutzungserscheinungen oder dergleichen zu schützen und gleichzeitig eine nochmalige Verbindung zur Otoplastik zu verstärken, wird diese anschließend versiegelt. Dadurch wird ein Eindringen und/oder Entweichen von Stoffen verhindert und ein möglicherweise scharfkantiges oder raues Material der Abdeckung mit einer glatten und kantenfreien Schicht überzogen bzw. überdeckt. Versiegeln ist dabei breit auszulegen, sodass damit auch ein Lackieren oder dergleichen mitumfasst ist.

Mit einem erfindungsgemäßen Verfahren kann beispielsweise eine Otoplastik zur Verwendung als Gehörschutz, Hörhilfe oder Miniaturkopfhörer hergestellt werden. Da nur eine sichtbare Fläche der Otoplastik durch das Aufkleben der Abdeckung verändert wird, kann mit einem solchen Verfahren eine Otoplastik relativ kostengünstig nach individuellen Wünschen hergestellt bzw. gestaltet werden. Ein zumindest teilweise in den Gehörgang einführbarer Bereich der Otoplastik wird bevorzugt so ausgebildet, dass insbesondere medizinische Ansprüche erfüllt werden. Beispielsweise soll die Otoplastik möglichst ohne allergische Reaktionen der Haut oder dergleichen über eine lange Zeit getragen werden können.

Vorteilhaft ist es, wenn zum Versiegeln der Abdeckung etwa mittig auf dieselbe eine auseinanderfließende Masse, insbesondere aus einem aushärtbaren Kunststoff, aufgebracht wird. Dies kann prinzipiell maschinell durchgeführt werden. Es ist jedoch günstig, wenn z. B. ein Tropfen von Hand aufgebracht wird, da dadurch auch das Auseinanderfließen des Tropfens beeinflusst bzw. gesteuert werden kann. Der Tropfen ist aus einem flüssigen oder zähflüssigen Kunststoff ausgebildet, welcher sich fließend über die Oberfläche der Abdeckung verteilt bzw. verteilt wird und schlussendlich eine Versiegelung ausbildet. Ferner wird hierfür ein aushärtbarer Kunststoff verwendet, welcher nach dem Auseinanderfließen bzw. Verlaufen mit z. B. UV-Licht ausgehärtet wird. Um eine Farbe bzw. ein Design der Abdeckung durch die Versiegelung durchsehen zu können, ist es günstig, wenn das Material des Tropfens bzw. die Versiegelung durchsichtig ist. Nach dem Aushärten der Versiegelung bzw. Versiegelungsschicht ist eine endgültige Form der Abdeckung bzw. Otoplastik hergestellt. Eine dadurch gebildete Oberfläche der Abdeckung weist bevorzugt eine gewölbte Form auf. Dadurch ist eine gesamte Oberfläche der Otoplastik im Wesentlichen ohne scharfe Kanten und Ecken ausgebildet, wodurch deren optisches Aussehen nochmals positiv hervorgehoben wird. Zusätzlich ist dadurch die Otoplastik verletzungsfrei in eine Ohrmuschel einführbar bzw. in einer solchen bewegbar. Die Wölbung der Versiegelung kann deren maximale Höhe dort aufweisen, wo der Tropfen ursprünglich aufgebracht wurde. Die Abdeckung ist mitsamt der Versiegelung an deren dickster Stelle etwa 1 mm bis 2 mm, insbesondere 1,3 mm bis 1,7 mm, bevorzugt etwa 1,5 mm dick.

Um die Abdeckung optimal an die Fläche der Otoplastik anzupassen, auf welche dieselbe geklebt wird, ist es von Vorteil, wenn aus dem digitalen dreidimensionalen Modell der Ohrmuschel ein digitales zweidimensionales Modell der sichtbaren Fläche berechnet wird. Dies wird bevorzugt digital mit einem Computer bzw. einer entsprechenden Software durchgeführt. Grundsätzlich ist es jedoch auch möglich, diesen Vorgang zumindest teilweise analog durchzuführen. Dazu wird ein Abdruck der Fläche der Otoplastik hergestellt, beispielsweise durch Aufbringen einer Farbe, welche anschließend auf ein Blatt Papier gestempelt bzw. gedruckt wird. Der gestempelte Abdruck kann dann gescannt und am Computer bearbeitet werden.

Es ist weiter vorteilhaft, wenn die Abdeckung dem zweidimensionalen Modell der sichtbaren Fläche entsprechend insbesondere mit einem Laser aus einem Werkstoff ausgeschnitten wird. Besonders bevorzugt wird das berechnete digitale zweidimensionale Modell in einem ersten Schritt so verkleinert, sodass dessen Umriss gleichmäßig um etwa 0,5 mm bis 1,5 mm, insbesondere 0,7 mm bis 1,3 mm, besonders bevorzugt etwa 1 mm, nach innen in Richtung Mittelpunkt des zweidimensionalen Modells versetzt wird. Dadurch ist es möglich, eine passgenaue Abdeckung zum Aufkleben auf die sichtbare Fläche zu erzeugen. Die Differenz zwischen der Fläche der Abdeckung und der sichtbaren Fläche der Otoplastik wird mit Versiegelungsmaterial aufgefüllt. Das Licht eines Lasers schneidet die Abdeckung exakt aus, ohne Konturen des Materials des Gegenstandes auszufransen oder dergleichen. Zudem kann mit einem Laser ein nahezu beliebiges Material annähernd mit einer gleich guten Schneidequalität geschnitten werden.

Zweckmäßig ist es, wenn die sichtbare Fläche vor einem Aufkleben der Abdeckung gereinigt und/oder poliert wird. Dadurch werden etwaige Unregelmäßigkeiten und/oder Unreinheiten auf der sichtbaren bzw. äußeren Fläche entfernt. Weiter ist dadurch ein möglichst gleichmäßiges Auftragen eines Klebers bzw. das Aufkleben der Abdeckung möglich. Besonders bevorzugt wird die sichtbare Fläche plan und frei von Kratzern, Rillen oder sonstigen Unebenheiten ausgebildet.

Es ist nicht zwingend, kann jedoch günstig sein, wenn die Otoplastik lackiert wird, um diese einerseits optisch ansprechend zu gestalten und andererseits resistenter gegenüber Abnutzungserscheinungen wie beispielsweise Kratzer auszubilden. Zur Lackierung wird insbesondere ein durchsichtiges Material verwendet.

Besonders günstig ist es, wenn ein Gehörschutz hergestellt wird. Ein solcher Gehörschutz kann beispielsweise von Sportlern, Musikern oder Arbeitern getragen werden. Diesen Personengruppen kann es wichtig sein, dass der Gehörschutz optisch ansprechend ausgebildet wird. Der Gehörschutz wird den individuellen Wünschen und Bedürfnissen eines Trägers entsprechend ausgebildet. Zum einen kann vom Träger ein Design und/oder Material der Abdeckung ausgewählt werden und zum anderen beispielsweise ein passendes Filterelement.

Wird ein Gehörschutz hergestellt, ist es von Vorteil, wenn zumindest eine Öffnung in die Abdeckung gebohrt wird, um eine Aufnahme von Umgebungsgeräuschen durch ein in der Otoplastik angeordnetes Filterelement zu ermöglichen. Das Filterelement kann dabei gleichzeitig mit der Otoplastik durch einen 3-D-Drucker gedruckt oder erst nach einem 3-D-Druck in die Otoplastik eingesetzt werden. Üblicherweise ist ein Gehörschutz nach einem Einsetzen des Filterelementes fertig hergestellt, wobei das Filterelement nach außen sichtbar angeordnet ist. Durch das Aufkleben der Abdeckung wird das Filterelement komplett abgedeckt, wobei im Filterelement ein Luftvolumen eingeschlossen wird. Dieses Luftvolumen erzeugt ein Resonanzsystem, durch welches ein Frequenzgang beeinflusst wird. Die Öffnung in der Abdeckung gewährleistet, dass trotz derselben Umgebungsgeräusche zum Filterelement gelangen können und von diesem aufgenommen bzw. gefiltert werden.

Zudem wird durch eine optimale Größe der Öffnung die Resonanzfrequenz so verändert, dass Filtereigenschaften möglichst positiv beeinflusst werden. Bevorzugt wird die Öffnung etwa rund bzw. kreisförmig und mit einem freien Durchmesser im Bereich von 1 mm bis 2 mm, insbesondere 1,4 mm bis 1,7 mm, bevorzugt etwa 1,6 mm ausgebildet. Dabei ist eine Wahl des freien Durchmessers der Öffnung besonders wichtig, um den Gehörschutz optimal auszubilden bzw. eine definierte Geräuschmenge auf das Filterelement auftreffen zu lassen. Es können auch zwei oder mehr Öffnungen zu einem Schalleintritt in die Abdeckung gebohrt werden, wobei der freie Durchmesser der Öffnungen jeweils verschieden groß ausgebildet sein kann. Es können beispielsweise zwei Bohrungen gebohrt werden, wobei eine erste davon einen freien Durchmesser im Bereich von 1 mm bis 1,5 mm und eine zweite einen freien Durchmesser im Bereich von 0,5 mm bis 1 mm aufweisen kann. Neben der Größe der zumindest einen Öffnung werden Eigenschaften des Filterelementes auch durch eine Dicke der Versiegelung bzw. deren Wölbung beeinflusst. Es kann folglich notwendig sein, eine Anzahl bzw. Größe einer bzw. mehrerer Öffnungen auf die Dicke bzw. einen Winkel der Wölbung der Versiegelung anzupassen.

Das weitere Ziel wird erreicht, wenn bei einer Otoplastik der eingangs genannten Art eine Abdeckung auf eine bei einem Tragen der Otoplastik in der Ohrmuschel sichtbare Fläche derselben geklebt und versiegelt ist.

Ein damit erzielter Vorteil ist insbesondere darin zu sehen, dass durch die Abdeckung eine optisch ansprechende Otoplastik geschaffen ist, welche vielfältig einsetzbar und langlebig ist. Da nur eine sichtbare Fläche der Otoplastik individualisierbar ist, ist die Otoplastik für mehrere Anwendungsbereiche nutzbar, wie beispielsweise als Hörhilfe, Gehörschutz oder Miniaturkopfhörer. Der zumindest teilweise in den Gehörgang einführbare Bereich der Otoplastik bleibt dem jeweiligen Anwendungsbereich entsprechend unverändert. Besonders günstig ist weiter, wenn eine großflächige Klebefläche vorgesehen ist. Dadurch ist ein lang anhaltender Zusammenschluss zwischen der Otoplastik und der Abdeckung gegeben. Die Abdeckung kann aus einem nahezu beliebigen Material hergestellt sein. Beispielsweise kann diese aus Holz, Papier, PVC-Folien bzw. Stoff hergestellt sein. Ein verwendeter Klebstoff ist dabei mit Vorteil an das Material der Abdeckung angepasst. Durch das Versiegeln bzw. die Versiegelung ist eine Haltbarkeit der Abdeckung an der Otoplastik nochmals verstärkt. Darüber hinaus ist die Abdeckung bzw. die Otoplastik gegenüber Abnutzungserscheinungen geschützt und frei von Ecken und Kanten ausgebildet. Die Otoplastik weist nicht nur ein ansprechendes Erscheinungsbild auf, sondern auch ohne eine Verletzungsgefahr im bzw. am Ohr verwendbar ist. Vorteilhaft ist es, wenn zum Versiegeln der Abdeckung eine auseinanderfließende Masse, z. B. ein oder mehrere Tropfen, etwa mittig auf die Abdeckung aufgebracht wird. Dies ist prinzipiell maschinell durchführbar. Es ist jedoch günstig, wenn der Tropfen von Hand aufgebracht wird, da dadurch auch das Auseinanderfließen des Tropfens beeinflussbar ist. Besonders bevorzugt ist ein solcher Tropfen aus einem beispielsweise mit UV-Licht aushärtbaren sowie durchsichtigen Kunststoff gebildet. Nach dem Aushärten der Versiegelung bzw. Versiegelungsschicht ist eine endgültige Form der Abdeckung bzw. Otoplastik hergestellt. Eine dadurch gebildete Oberfläche der Abdeckung ist bevorzugt nicht eben bzw. flach, sondern mit einer gewölbten Form ausgebildet.

Es kann günstig sein, wenn ein Filterelement in die Otoplastik eingesetzt ist. Dadurch ist die Otoplastik als Gehörschutz ausgebildet. Das Filterelement wird bevorzugt vor einem Aufbringen bzw. Kleben der Abdeckung in die Otoplastik eingesetzt. Beim Aufkleben der Abdeckung ist das Filterelement von der Klebefläche ausgenommen, um eine Wirkung desselben nicht zu beeinträchtigen.

Es ist dazu weiter zweckmäßig, wenn zumindest eine Öffnung in der Abdeckung vorgesehen ist, um eine Aufnahme von Umgebungsgeräuschen durch das Filterelement zu ermöglichen. Zweckmäßig ist es, wenn die Öffnung direkt über dem Filterelement angeordnet ist, wobei das Filterelement von einer Klebefläche ausgenommen ist. Das Filterelement ist etwa zylinderförmig ausgebildet und weist eine etwa runde Oberfläche auf, welche in die sichtbare Fläche der Otoplastik eingebettet ist. Ein Durchmesser einer obersten Schicht des Filterelementes kann dabei größer sein als ein restlicher Körper des Filterelementes. Ein freier Durchmesser der bevorzugt rund ausgebildeten Öffnung kann beispielsweise 1 mm bis 2 mm, insbesondere 1,4 mm bis 1,7 mm, bevorzugt etwa 1,6 mm groß sein. Die Öffnung kann jedoch auch mit einem rechteckigen oder anders polygonalen Querschnitt ausgebildet sein. Darüber hinaus können auch zwei oder mehr Öffnungen vorgesehen sein, welche jeweils einen verschieden großen freien Durchmesser aufweisen können.

Vorteilhaft kann ein Verhältnis eines freien Durchmessers der Öffnung zu einem Durchmesser einer Oberfläche des Filterelementes zumindest 1:4, insbesondere zumindest 1:5, bevorzugt zumindest 1:6 sein. Dabei ist ein etwa runder Querschnitt der Öffnung und des Filterelementes angenommen. Bei allen denkbaren Querschnitten der Öffnung und des Filterelementes ist es günstig, wenn ein Verhältnis einer Fläche der Öffnung zu der Oberfläche des Filterelementes zumindest 1:16, insbesondere zumindest 1:25, bevorzugt zumindest 1:36 ist. Durch das Aufkleben der Abdeckung auf das Filterelement ist dieses komplett abgedeckt, wobei dadurch im Filterelement ein Luftvolumen eingeschlossen ist. Dieses Luftvolumen erzeugt ein Resonanzsystem, welches einen Frequenzgang beeinflusst. Die Öffnung in der Abdeckung gewährleistet, dass trotz derselben Umgebungsgeräusche zum Filterelement gelangen, welches diese aufnimmt bzw. filtert. Zudem wird durch eine optimale Größe der Öffnung die Resonanzfrequenz so verändert, dass dadurch Filtereigenschaften möglichst positiv beeinflusst sind. Neben der Größe der zumindest einen Öffnung sind Eigenschaften des Filterelementes auch durch eine Dicke der Versiegelung bzw. deren Wölbung beeinflusst. Somit kann es notwendig sein, eine Anzahl bzw. Größe einer bzw. mehrerer Öffnungen auf die Dicke bzw. einen Winkel der Wölbung der Versiegelung anzupassen.

Eine Verwendung einer erfindungsgemäßen Otoplastik erfolgt mit Vorteil als Gehörschutz, welcher insbesondere optisch ansprechend ausgebildet ist.

Weitere Merkmale, Vorteile und Wirkungen ergeben sich aus den nachfolgend dargestellten Ausführungsbeispielen. In den Zeichnungen, auf welche dabei Bezug genommen wird, zeigen:
- Fig. 1: eine Draufsicht auf eine Otoplastik;
- Fig. 2: eine erfindungsgemäße Otoplastik;
- Fig. 3: eine weitere Ansicht einer erfindungsgemäßen Otoplastik gemäß Fig. 2;
- Fig. 4: eine Explosionsdarstellung einer erfindungsgemäßen Otoplastik.

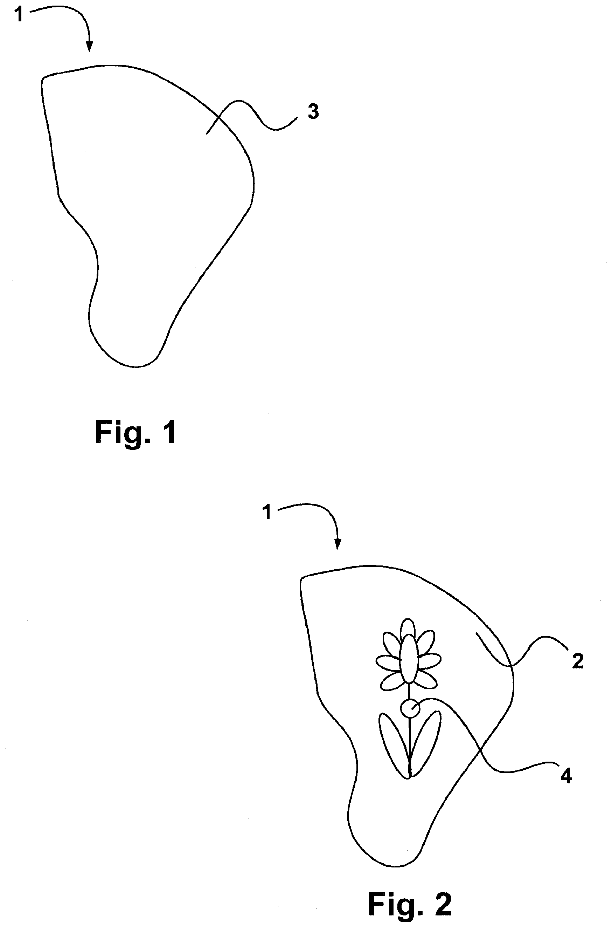

Fig. 1 zeigt eine Otoplastik 1. Dabei ist eine Draufsicht der Otoplastik 1 gezeigt, wobei eine Fläche 3 dargestellt ist, welche bei einem Tragen der Otoplastik 1 in der Ohrmuschel nach außen hin sichtbar ist. Zur Herstellung der Otoplastik 1 wird in einem ersten Schritt ein dreidimensionales Modell einer Ohrmuschel erstellt. Dies kann prinzipiell durch ein Abscannen der Ohrmuschel mit beispielsweise Laserlicht durchgeführt werden. Um ein möglichst exaktes Abbild der Ohrmuschel zu erzeugen, ist es jedoch günstig, dieses mittels einer Abformmasse abzuformen, sodass eine Vorlage zur Herstellung der Otoplastik 1 bereitgestellt wird. Diese Vorlage wird anschließend von einem 3-D-Scanner aufgenommen und ein digitales dreidimensionales Bild davon erzeugt. Das digitale Bild wird mit einem Computer bzw. einer entsprechenden Software nachbearbeitet und optimiert, wonach dieses Abbild des Ohres bzw. der Ohrmuschel mit einem 3-D-Drucker gedruckt wird. Bei einem erfindungsgemäßen Verfahren wird auf die nach außen plane Fläche 3 eine Abdeckung 2 geklebt, welche anschließend versiegelt wird.

Eine erfindungsgemäße Otoplastik 1 mit einer Abdeckung 2 ist in Fig. 2 gezeigt, wobei die Abdeckung 2 aus einem beliebigen Material wie beispielsweise Papier, Holz, Kunststoff oder Metall hergestellt sein kann. Weiter kann das Material ein beliebiges Motiv bzw. Design aufweisen. Um eine langlebige Otoplastik 1 ohne Anfälligkeiten für Abnutzungserscheinungen herzustellen, wird diese versiegelt. Dazu wird etwa mittig auf die Fläche der Otoplastik ein Tropfen eines eine Versiegelung 6 (in Fig. 2 nicht dargestellt) herstellenden Materials aufgebracht. Besonders bevorzugt wird hierzu ein aushärtbarer, zähflüssiger oder flüssiger Kunststoff verwendet, welcher beispielsweise unter UV-Licht ausgehärtet wird. Günstig ist es, wenn der Tropfen händisch aufgebracht wird, sodass ein Auseinanderfließen desselben kontrolliert werden kann. Die Versiegelung 6 ist zudem bevorzugt durchsichtig ausgebildet, sodass ein Motiv bzw. ein Material der Abdeckung 2 durch diese hindurch sichtbar bleibt. Nach dem Aushärten der Versiegelung 6 ist eine endgültige Form der Abdeckung 2 bzw. Otoplastik 1 hergestellt. Eine durch die Versiegelung 6 gebildete Oberfläche der Abdeckung 2 weist bevorzugt eine gewölbte Form auf. Dadurch ist eine gesamte Oberfläche der Otoplastik 1 im Wesentlichen ohne scharfe Kanten und Ecken ausgebildet, wodurch deren optisches Aussehen nochmals positiv hervorgehoben wird. Zusätzlich wird dadurch das Ohr nicht durch scharfe Kanten oder dergleichen verletzt. Die Wölbung der Versiegelung 6 kann deren maximale Höhe dort aufweisen, wo der Tropfen ursprünglich aufgebracht wurde.

Um eine Abdeckung 2 herzustellen, welche passgenau auf die sichtbare Fläche 3 der Otoplastik 1 geklebt wird, wird aus dem digitalen dreidimensionalen Modell der Ohrmuschel ein digitales zweidimensionales Modell der sichtbaren Fläche 3 berechnet. Besonders bevorzugt wird ein Modell mit verkleinerten Konturen im Vergleich zur Fläche 3 erstellt, wobei diese um etwa 0,5 mm bis 1,5 mm, insbesondere 0,7 mm bis 1,3 mm, besonders bevorzugt etwa 1 mm, nach innen in Richtung Mittelpunkt versetzt werden. Die Abdeckung 2 wird dann gemäß diesem Modell beispielsweise mit einem Laser aus einem Werkstoff ausgeschnitten und auf die Fläche 3 der Otoplastik 1 geklebt. Um eine optimale Verbindung zwischen der Fläche 3 und der Abdeckung 2 herzustellen, wird ein Kleber großflächig aufgetragen. Zudem ist es günstig, wenn die Fläche 3 vor einem Aufkleben der Abdeckung 2 gereinigt und/oder poliert wird, um eine möglichst plane Oberfläche frei von Kratzern, Unebenheiten oder sonstigen Unregelmäßigkeiten zu erzeugen. Darüber hinaus kann die Otoplastik 1 lackiert werden.

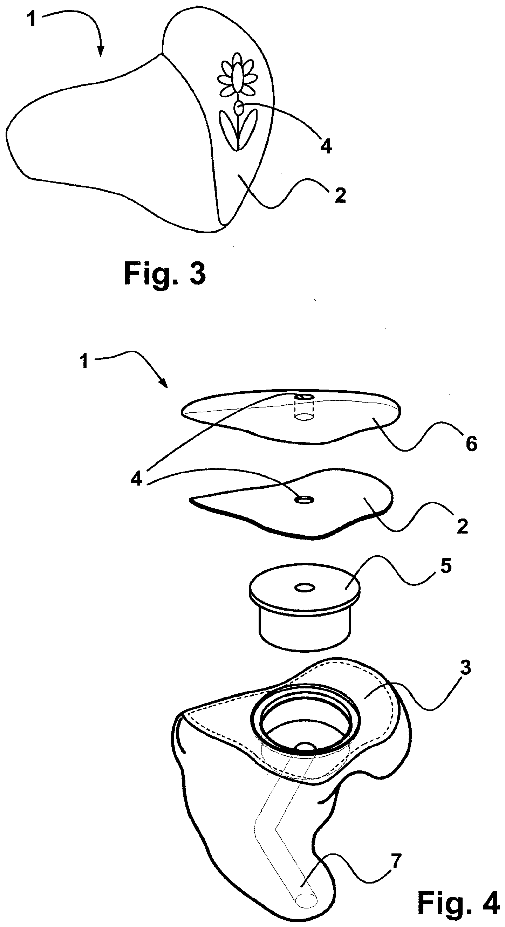

Insbesondere kann mit dem erfindungsgemäßen Verfahren ein Gehörschutz hergestellt werden. Hierbei wird dem 3-D-Druck der Otoplastik 1 ein Filterelement 5 in dieselbe eingesetzt, welches Umgebungsgeräusche filtern soll. Die Abdeckung 2 wird so auf die Fläche 3 aufgeklebt, dass das Filterelement 5 von dieser komplett abgedeckt wird. Um trotzdem ein Auftreffen von zu filternden Geräuschen auf das Filterelement 5 sicherzustellen, wird zumindest eine Öffnung 4 in die Abdeckung 2 gebohrt. In Fig. 2 ist die Öffnung 4 dargestellt, wobei das Filterelement 5 nicht gezeigt ist. Eine Otoplastik 1 mit Filterelement 5 ist in weiterer Folge in Fig. 4 gezeigt.

In Fig. 3 ist eine weitere Ansicht der Otoplastik 1 gemäß Fig. 2 gezeigt. Bevorzugt wird die Öffnung 4 etwa rund bzw. kreisförmig ausgebildet. Dabei ist ein freier Durchmesser bzw. eine Fläche der Öffnung 4 besonders wichtig, um den Gehörschutz optimal auszubilden bzw. eine definierte Geräuschmenge auf das in Fig. 3 nicht dargestellte Filterelement 5 auftreffen zu lassen. Es können auch zwei oder mehr Öffnungen 4 zu einem Schalleintritt in die Abdeckung 2 gebohrt werden, wobei der freie Durchmesser der Öffnungen 4 jeweils verschieden groß sein kann. Es können beispielsweise zwei Öffnungen 4 in die Abdeckung 2 gebohrt werden, wobei eine erste davon einen freien Durchmesser im Bereich von 1 mm bis 1,5 mm und eine zweite einen freien Durchmesser im Bereich von 0,5 mm bis 1 mm aufweist. Die Öffnungen 4 werden beabstandet zueinander angeordnet, wobei eine Größe eines Abstandes zu einer auf das Filterelement 5 auftreffenden Geräuschmenge beitragen kann.

Fig. 4 zeigt eine Explosionsdarstellung einer erfindungsgemäßen Otoplastik 1, welche zur Verwendung als Gehörschutz hergestellt wird. Es ist ein Filterelement 5 gezeigt, welches im Wesentlichen zylinderförmig ausgebildet und in der Otoplastik 1 eingesetzt ist. Das Filterelement 5 weist eine etwa runde Oberfläche auf, welche in eine sichtbare Fläche 3 der Otoplastik 1 eingebettet ist. Ein Durchmesser einer obersten Schicht ist größer als ein restlicher Körper des Filterelementes 5, um dieses positionsstabil in der Otoplastik 1 platzieren zu können. Auf die Fläche 3 ist eine Abdeckung 2 geklebt, wobei ein Bereich des Filterelementes 5 von einer Klebefläche ausgenommen ist. In Fig. 4 ist dabei durch die gestrichelte Linie auf der Fläche 3 angedeutet, dass eine Fläche der Abdeckung 2 etwas kleiner als die Fläche 3 ist. Weiter ist eine Öffnung 4 in die Abdeckung 2 gebohrt, sodass Umgebungsgeräusche zum Filterelement 5 gelangen können. In Fig. 4 ist zudem eine Versiegelung 6 gezeigt, welche die Abdeckung 2 versiegelt und eine gewölbte Form aufweist. Auch die Versiegelung 6 weist eine Öffnung 4 auf, wobei diese erst nach dem Kleben und Versiegeln der Abdeckung 2 gleichzeitig durch die Abdeckung 2 und die Versiegelung gebohrt wird. Als Versiegelung 6 wird ein aushärtbarer, durchsichtiger Kunststoff verwendet, welcher z. B. als ein oder mehrere Tropfen einer auseinanderfließenden Masse auf die Abdeckung 2 aufgebracht wird.

In Fig. 4 ist weiter ein Kanal 7 gezeigt, durch welchen eine definierte Geräuschmenge über das Filterelement 5 durch die Otoplastik 1 auf den Gehörgang einer den Gehörschutz tragenden Person trifft bzw. geleitet wird. Der Kanal 7 reicht von einem unteren Ende des Filterelementes 5, welches innerhalb der Otoplastik 1 gelagert ist, bis zu einem innenseitigen Ende der Otoplastik 1, welche in Richtung des Gehörganges der den Gehörschutz tragenden Person zeigt.

Neben der Verwendung als Gehörschutz kann die Otoplastik 1 auch als Hörhilfe, Miniaturkopfhörer oder im Ohr tragbarer Miniaturcomputer verwendet werden. Hierzu können eine entsprechende Elektronik und/oder andere notwendige Bauteile in der Otoplastik 1 platziert werden. Die sichtbare Fläche 3 wird dabei stets mit der Abdeckung 2 verklebt, sodass ein gewünschtes Material und/oder Motiv nach außen hin sichtbar ist. Das Material und/oder Motiv kann von einer die Otoplastik 1 tragenden Person individuell ausgewählt werden.

## Patentansprüche

1. Verfahren zum Herstellen einer Otoplastik (1), wobei ein digitales dreidimensionales Modell einer Ohrmuschel erstellt wird, gemäß welchem die Otoplastik (1) mit einem 3-D-Drucker gedruckt wird, **dadurch gekennzeichnet, dass** ein Gehörschutz hergestellt wird, wobei eine Abdeckung (2) auf eine bei einem Tragen der Otoplastik (1) in der Ohrmuschel sichtbare Fläche (3) derselben geklebt und die Abdeckung (2) danach mit einer glatten und kantenfreien Schicht versiegelt wird, wobei durch das Aufkleben der Abdeckung (2) ein Filterelement (5) komplett abgedeckt wird, wobei dadurch im Filterelement (5) ein Luftvolumen eingeschlossen wird, wobei durch dieses Luftvolumen ein Resonanzsystem erzeugt wird, durch welches ein Frequenzgang beeinflusst wird, und wobei zumindest eine Öffnung (4) in die Abdeckung (2) gebohrt wird, um eine Aufnahme von Umgebungsgeräuschen durch das in der Otoplastik (1) angeordnete Filterelement (5) zu ermöglichen.

2. Verfahren nach Anspruch 1, **dadurch gekennzeichnet, dass** zum Versiegeln der Abdeckung (2) etwa mittig auf dieselbe eine auseinanderfließende Masse, insbesondere aus einem aushärtbaren Kunststoff, aufgebracht wird.

3. Verfahren nach Anspruch 1 oder 2, **dadurch gekennzeichnet, dass** aus dem digitalen dreidimensionalen Modell der Ohrmuschel ein digitales zweidimensionales Modell der sichtbaren Fläche (3) berechnet wird.

4. Verfahren nach Anspruch 3, **dadurch gekennzeichnet, dass** die Abdeckung (2) dem zweidimensionalen Modell der sichtbaren Fläche (3) entsprechend insbesondere mit einem Laser aus einem Werkstoff ausgeschnitten wird.

5. Verfahren nach einem der Ansprüche 1 bis 4, **dadurch gekennzeichnet, dass** die sichtbare Fläche (3) vor dem Aufkleben der Abdeckung (2) gereinigt und/oder poliert wird.

6. Verfahren nach einem der Ansprüche 1 bis 5, **dadurch gekennzeichnet, dass** die Otoplastik (1) lackiert wird.

7. Otoplastik (1) zum teilweisen Einführen in einen Gehörgang und/oder Tragen in einer Ohrmuschel, wobei die Otoplastik (1) mit einem 3-D-Drucker gedruckt und als Gehörschutz ausgebildet ist, **dadurch gekennzeichnet, dass** eine Abdeckung (2) auf eine bei einem Tragen der Otoplastik (1) in der Ohrmuschel sichtbare Fläche (3) derselben geklebt und mit einer glatten und kantenfreien Schicht versiegelt ist, wobei durch die aufgeklebte Abdeckung (2) ein Filterelement (5) komplett abgedeckt ist, um dadurch im Filterelement (5) ein Luftvolumen einzuschließen und durch dieses Luftvolumen ein Resonanzsystem zu erzeugen, durch welches ein Frequenzgang beeinflusst wird, wobei zumindest eine Öffnung (4) in die Abdeckung (2) gebohrt ist, um eine Aufnahme von Umgebungsgeräuschen durch das in der Otoplastik (1) angeordnete Filterelement (5) zu ermöglichen.

8. Otoplastik (1) nach Anspruch 7, **dadurch gekennzeichnet, dass** ein Filterelement (5) in die Otoplastik (1) eingesetzt ist.

9. Otoplastik (1) nach Anspruch 8, **dadurch gekennzeichnet, dass** zumindest eine Öffnung (4) in der Abdeckung (2) vorgesehen ist, um eine Aufnahme von Umgebungsgeräuschen durch das Filterelement (5) zu ermöglichen.

10. Otoplastik (1) nach Anspruch 9, **dadurch gekennzeichnet, dass** ein Verhältnis eines freien Durchmessers der Öffnung (4) zu einem Durchmesser einer Oberfläche des Filterelementes (5) zumindest 1:4, insbesondere zumindest 1:5, bevorzugt zumindest 1:6 ist.

## Claims

1. A method for producing an earmould (1), wherein a digital, three-dimensional model of an external ear is created, on the basis of which the earmould (1) is printed with a 3D printer, **characterized in that** a hearing protector is produced, wherein a covering (2) is glued to a surface (3) of the earmould (1) which is visible when said earmould is worn in the external ear, and the covering (2) is subsequently sealed with a smooth layer having no edges, wherein the gluing of the covering (2) has the effect of completely covering a filter element (5), so that in turn a volume of air is consequently enclosed inside the filter element, wherein said volume of air creates a resonant system by which a frequency response is influenced, and wherein at least one opening (4) is drilled into the covering (2) to enable ambient noises to be absorbed by the filter element (5) which is arranged inside the earmould (1) .

2. The method according to claim 1, **characterized in that** a diffluent mass consisting in particular of a curable plastic is applied to the covering (2) roughly in the middle thereof in order to seal it.

3. The method according to claim 1 or 2, **characterized in that** a digital two-dimensional model of the visible surface (3) is calculated from the digital three-dimensional model of the external ear.

4. The method according to claim 3, **characterized in that** the covering (2) is cut out of a material particularly with a laser correspondingly to the two-dimensional model of the visible surface (3).

5. The method according to any one of claims 1 to 4, **characterized in that** the visible surface (3) is cleaned and/or polished before the covering (2) is glued on.

6. The method according to any one of claims 1 to 5, **characterized in that** the earmould (1) is lacquered.

7. An earmould (1) designed to be partially inserted in an ear canal and/or worn in an external ear, wherein the earmould (1) is printed with a 3D printer and designed as a hearing protector, **characterized in that** a covering (2) is glued to a surface (3) of the earmould (1) which is visible when said earmould is worn in the external ear, and is sealed with a smooth layer having no edges, wherein the gluing of the covering (2) has the effect of completely covering a filter element (5), in order to thus enclose a volume of air inside the filter element (5) and create a resonant system with this volume of air, whereby a frequency response is influenced, wherein at least one opening (4) is drilled into the covering (2) to enable ambient noises to be absorbed by the filter element (5) which is arranged inside the earmould (1) .

8. The earmould (1) according to claim 7, **characterized in that** a filter element (5) is inserted in the earmould (1).

9. The earmould (1) according to claim 8, **characterized in that** at least one opening (4) is provided in the covering (2) to enable ambient noises to be absorbed by the filter element (5).

10. The earmould (1) according to claim 9, **characterized in that** a ratio between a free diameter of the opening (4) and a diameter of a surface of the filter element (5) is equal to at least 1:4, particularly at least 1:5, preferably at least 1:6.

## Revendications

1. Procédé de production d'un otoplastique (1), dans lequel est établi un modèle tridimensionnel numérique d'un pavillon d'oreille selon lequel l'otoplastique (1) est imprimé avec une imprimante 3D, **caractérisé en ce qu'**une protection d'oreille est réalisée, un recouvrement (2) étant collé dans le pavillon d'oreille sur une surface (3) visible de celui-ci lors du port de l'otoplastique (1) et le recouvrement (2) étant ensuite scellé avec une couche lisse et sans arête, un élément filtrant (5) étant complètement recouvert par le collage du recouvrement (2), un volume d'air étant ainsi renfermé dans l'élément filtrant (5), étant créé par ce volume d'air un système de résonance par lequel une réponse de fréquence est influencée, et au moins une ouverture (4) étant percée dans le recouvrement (2) pour permettre une absorption des bruits environnants par l'élément filtrant (5) disposé dans l'otoplastique (1).

2. Procédé selon la revendication 1, **caractérisé en ce que**, pour sceller le recouvrement (2), approximativement au centre, une masse s'étalant, en particulier composée d'une matière plastique durcissable, est appliquée.

3. Procédé selon la revendication 1 ou 2, **caractérisé en ce que**, à partir du modèle tridimensionnel numérique du pavillon d'oreille, un modèle bidimensionnel numérique de la surface visible (3) est calculé.

4. Procédé selon la revendication 3, **caractérisé en ce que** le recouvrement (2) est découpé en fonction du modèle bidimensionnel de la surface visible (3), en particulier avec un laser, dans un matériau.

5. Procédé selon une des revendications 1 à 4, **caractérisé en ce que** la surface visible (3) set nettoyée et/ou polie avant le collage du recouvrement (2).

6. Procédé selon une des revendications 1 à 5, **caractérisé en ce que** l'otoplastique (1) est verni.

7. Otoplastique (1) destiné à une insertion partielle dans un conduit auditif et/ou à un port dans un pavillon d'oreille, l'otoplastique (1) étant imprimé avec une imprimante 3D et réalisé sous forme d'une protection d'oreille, **caractérisé en ce qu'**un recouvrement (2) est collé dans le pavillon d'oreille sur une surface visible (3) de celui-ci lors d'un port de l'otoplastique (1) et scellé avec une couche lisse et sans arête, un élément filtrant (5) étant complètement couvert par le recouvrement collé (2) pour ainsi renfermer un volume d'air dans l'élément filtrant (5) et créer grâce à ce volume d'air un système de résonance qui influence une réponse de fréquence, au moins une ouverture (4) étant percée dans le recouvrement (2) pour permettre une absorption de bruits environnants par l'élément filtrant (5) disposé dans l'otoplastique (1).

8. Otoplastique (1) selon la revendication 7, **caractérisé en ce qu'**un élément filtrant (5) est inséré dans l'otoplastique (1).

9. Otoplastique (1) selon la revendication 8, **caractérisé en ce qu'**il est prévu dans le recouvrement (2) au moins une ouverture (4) pour permettre une absorption de bruits environnants par l'élément filtrant (5).

10. Otoplastique (1) selon la revendication 9, **caractérisé en ce qu'**un rapport entre un diamètre libre de l'ouverture (4) et un diamètre d'une surface de l'élément filtrant (5) s'élève à au moins 1:4, en particulier au moins 1:5, de préférence au moins 1:6.
